# EUROPÄISCHE PATENTSCHRIFT

(11) **EP 2 470 265 B1**
(45) Veröffentlichungstag und Bekanntmachung des Hinweises auf die Patenterteilung: **17.12.2014**
(21) Anmeldenummer: 10743184.3
(22) Anmeldetag: 20.08.2010
(51) Int. Cl.: A61N 2/02

(54) **VORRICHTUNG ZUR STIMULIERUNG AUTOREGULATIVER LOKALER UND ÜBERGEORDNETER MECHANISMEN DER HOMÖOSTASE DES ORGANISMUS**
DEVICE FOR STIMULATING AUTOREGULATIVE LOCAL AND SUPERORDINATE MECHANISMS OF THE HOMEOSTASIS IN THE ORGANISM
DISPOSITIF DE STIMULATION DE MÉCANISMES AUTORÉGULATEURS LOCAUX ET SUPÉRIEURS DE L'HOMÉOSTASIE DE L'ORGANISME

(30) Priorität: 25.08.2009 EP 09168634
(43) Veröffentlichungstag der Anmeldung: 04.07.2012
(73) Patentinhaber: BEMER International AG, 9495 Triesen (LI)
(72) Erfinder: Gleim, Peter, FL-9495 Schliessa (LI); KLOPP, Rainer, 16348 Wandlitz (DE)
(74) Vertreter: Gulde & Partner
(86) Internationale Anmeldenummer: PCT/EP2010/062166
(87) Internationale Veröffentlichungsnummer: WO 2011/023635

(56) Entgegenhaltungen:
- WO-A1-00/76582
- WO-A1-2008/025731
- WO-A1-2009/090440
- WO-A2-2008/127011
- DE-A1- 10 237 519

## Beschreibung

Die Erfindung betrifft eine Vorrichtung zur Stimulierung autoregulativer lokaler und übergeordneter Mechanismen der Homöostase während der Schlafphase und der Ruhe- bzw. Entspannungsphase des Organismus auf Basis eines pulsierenden elektromagnetischen Feldes.

Es ist bereits bekannt, die Mikrozirkulation durch elektromagnetische Impulse zu beeinflussen.

Aus der EP 0 995 463 ist eine Vorrichtung bekannt, mittels derer biologische Abläufe im menschlichen Körper durch pulsierende elektromagnetische Felder beeinflusst werden, um insbesondere die O₂-Utilisation zu erhöhen und Stoffwechselvorgänge anzuregen. Die Einzelimpulse können dabei einer formelmäßig dargestellten Funktion folgen.

Die WO 2008/025731 beschreibt eine Vorrichtung zur Erzeugung eines gepulsten elektromagnetischen Feldes mit periodischen Impulsen mit an- und absteigenden Hüllkurven in Abhängigkeit von bestimmten Messdaten der Mikrozirkulation des Blutes.

In der WO 00/76582 wird die lonentransportaktivierung durch Zellmembranen und Kapillarwände mittels niedriggepulsten elektromagnetischen Feldern und stufenförmigen Impulsen beschrieben.

Die WO 2008/127011 beschreibt eine Vorrichtung zur Magnetfeldtherapie mit stufenförmigen Frequenzverläufen mit Frequenzen von 8,7 - 250 Hz und Pulsen im Millisekundenbereich.

Aus der WO 2009/090440 ist eine Diabetesbehandlung mittels gepulstem Magnetfeld bekannt, wobei nur allgemein beschrieben ist, daß die Intensität weniger als 50 µT und die Frequenz 1 Hz bis 1 MHz betragen kann.

Das Gerät zur Magnetfeldtherapie aus DE 102 37 519 A1 erzeugt Frequenzkomponenten von 300 - 1000 Hz mit überlagerten Frequenzen von 2 - 32 Hz, wobei die Intensität von 1,5 bis 150 µT gesteigert werden kann.

Der Erfindung liegt die Aufgabe zugrunde, eine Vorrichtung bereitzustellen, mit der höhere Regenerations- und Restitutionseffekte im Bereich der Mikrozirkulation während der Schlafphase und der Relaxationsphase des Organismus erreicht werden, die der Umverteilung des reduzierten Herz-Minuten-Volumens in der Schlaf- bzw. Relaxationsphase Rechnung trägt. Dies betrifft neben Funktionssteigerungen in der Mikrozirkulation auch den Lymphstrom und vor allem das Immunsystem. Erfindungsgemäß umfasst die Vorrichtung einen Impulsgenerator, ein Steuergerät und eine Felderzeugungsvorrichtung, die zusammen ein pulsierendes elektromagnetisches Feld erzeugen, wobei Impulsfolgen mit bestimmten Höhen, bestimmten Abständen und bestimmten Frequenzen die Pulsation des Feldes beeinflussen. Damit können Stimulierungseffekte für autoregulative Mechanismen der Homöostase des Organismus hervorgerufen werden.

Bei der Überlegung, welches morphologische und funktionelle System des Organismus repräsentative Aussagen zu therapierelevanten Wirkungen einer Behandlungsmethode erlaubt, muss dem Transportorgan Blut eine herausragende Bedeutung zugeordnet werden. Die biologische Aufgabe des Organes Blut besteht hauptsächlich in seinen Leistungen zur Homöostase, d.h. der Aufrechterhaltung einer Konstanz des "inneren Milieus" im Rahmen des Zusammenwirkens verschiedener Steuer- und Regelungsvorgänge bei der Realisierung von Fließgleichgewichten sowie seiner Leistungen bei Immunreaktionen.

Die wichtigsten "konstanten Merkmale" des Organismus sind unter physiologischen Bedingungen:
- Wassergehalt
- Zusammensetzung der Körperflüssigkeiten. Blutkonstanten: z.B. pH = 7,3, Gehalt an mineralischen Ionen (Natrium, Kalium, Magnesium, Eisen, Chlorid, Phosphat, Hydrogenkarbonat, Proteingehalt), auch die Glukose-Konzentrationen werden auf einen nur wenig schwankenden Pegel eingeregelt. Erhaltung des Blutvolumens (transkapillärer Flüssigkeitsaustausch, Blutungsstillung, Gerinnung)
- Körpertemperatur des (systemischen) mittleren arteriellen Blutdruckes.

Der lebende Organismus ist ein multistabiles System, in dem verschiedene morphologische Einheiten zur Gewährleistung der Homöostase funktionell durch das Organ Blut verknüpft sind. Diese Einheiten sind äußere Atmung und Stoffwechsel mit dem Herz-Lungen-Kreislauf sowie Nierenfunktion mit der Blutdruckregulierung.

Beim komplizierten Zusammenwirken von Steuer- und Regelungsvorgängen im Organismus mit dem Ziel einer Aufrechterhaltung von Homöostase sind alle Teile des Organismus so miteinander verbunden, dass Änderungen in einem der Teilsysteme (Gewebe, Organ, Organsystem) auf andere Teilsysteme wirken und diese veranlasst, einen neuen Gleichgewichtszustand herzustellen, wobei die Kopplung nerval und durch den Blutkreislauf, u.a. durch die Mikrozirkulation, erfolgt.

Der Funktionszustand eines Organes wird wesentlich durch den Funktionszustand seiner Mikrozirkulation bestimmt. Es ist heute allgemein akzeptiert, dass die meisten Funktionsstörungen bzw. krankhaften Zustände der Organe durch Mikrozirkulationsstörungen wenn nicht sogar ausgelöst, so doch zumindest in ihrem Verlauf determiniert sind. Mikrozirkulationsstörungen entstehen oft im Gefolge von Makrozirkulationsstörungen und können nach und nach eine eigene Dynamik entfalten, die unabhängig vom makrozirkulatorischen Geschehen den Krankheitsverlauf wesentlich beeinflusst oder gar dominiert. Ohne entsprechende Beteiligung der Mikrozirkulation, d.h. der Transportprozesse im Bereich der Mikrogefäße, sind keine Funktionssteigerungen, Abheilungs- oder Restitutionsvorgänge oder Regenerationsvorgänge in den Organen möglich. Wenn überhaupt, so können bei restriktiver Mikrozirkulation bestenfalls vorübergehend in einem geringen Ausmaß Störungs- oder KrankheitsSymptome beeinflusst werden.

Der morphologisch-funktionelle Bereich der Mikrozirkulation lässt daher therapierelevante und prophylaktisch relevante Wirkungen besonders klar erkennen.

Mit der erfindungsgemäßen Vorrichtung wird in erster Linie das Ziel verfolgt, bestimmte Effekte im Bereich der Mikrozirkulation des Blutes hervorzurufen und durch wissenschaftlich bewährte Messmethoden und Messkriterien sichtbar und nachprüfbar zu machen.

Die erfindungsgemäße Vorrichtung, die einen Impulsgenerator, ein Steuergerät und eine Felderzeugungseinrichtung umfasst, wobei der Impulsgenerator so ausgestaltet ist, dass er zusammen mit dem Steuergerät und der Felderzeugungseinrichtung ein pulsierendes elektromagnetisches Feld erzeugt durch Abgabe von Impulsfolgen, wobei die Vorrichtung gekennzeichnet ist durch eine Impulsfolge, die die Folgen 1 bis 5 einschließt:
Folge 1: 0 µT für 1-3 Sekunden,
Folge 2: 3-12 µT Grundsignal für 12-16 Sekunden,
Folge 3: 30-150 µT Zusatzsignal für 100-200 Millisekunden,
Folge 4: 8- bis 10-malige Wiederholung der Folgen 2 und 3,
Folge 5: 0 µT für 1-3 Sekunden
mit folgenden Maßgaben
   (1) die Frequenz für das Grundsignal und das Zusatzsignal ist gleich und liegt im Bereich von 8 bis 15 Hz;
   (2) die Impulsfolgen sind derart eingerichtet, dass sie in mehrfacher Wiederholung auftreten und dabei die Steuerzeit aller mehrfach wiederholten Impulsfolgen auf wenigstens 3,5 Stunden innerhalb eines Zeitraumes von 7-9 Stunden bemessen ist;
   (3) die Amplituden der Einzelimpulse folgen einer Exponentialfunktion bogenförmig oder an- und absteigenden Hüllkurven mit harmonischem oder anharmonischem Verlauf.
Das Grundsignal kann durch 3 bis 8 Pausen von 1 bis 3 Sekunden mit 0 µT unterbrochen und damit verkürzt sein.

Die Steuerzeit kann so bemessen sein, dass sie bis zu 8 Stunden beträgt und mehrfach wiederholte Impulsfolgen in dieser Zeit durchgehend zulässt. In einer weiteren Ausführungsform der Erfindung kann die Steuerzeit auch so bemessen sein, dass sie Impulsfolgen nur für 2 Stunden und nochmals 2 Stunden nach einer 4-stündigen Pause ohne Signale (0 µT) zulässt. In einer weiteren Ausführungsform der Erfindung kann die Steuerzeit auch so bemessen sein, dass sie Impulsfolgen in 20- bis 40-minütigen Intervallen mit entsprechenden Pausen ohne Signale (0 µT) dazwischen zulässt.

Die Felderzeugungseinrichtung ist eine großflächige Spulenmatte zur Ganzkörperbehandlung, vorzugsweise in den Abmaßen 60 - 80 cm x 150 - 190 cm. Bei einer solchen Spulenmatte können mehrere elektromagnetische Einzelspulen in gleichmäßiger oder ungleichmäßiger Verteilung über die Gesamtfläche der Matte zur Erzeugung eines flächenhaften Magnetfeldes angeordnet sein. Je nach Größe der Matte kann diese auch nur eine einzige Spule enthalten.

Die Einzelspulen sind vorteilhaft als flächeninhaltsgleiche Leiterschleifen oder als Leiterschleifen mit unterschiedlichen Flächen oder als beide Varianten zusammen ausgebildet.

Der Begriff "Grundsignal" im Sinne der Erfindung bedeutet, dass die Maxima der Einzelschwingungen innerhalb der vorgegebenen Zeiteinheit im wesentlichen gleich groß sind und den vorgegebenen Intensitätswert nicht überschreiten, d.h. beispielsweise nicht höher als 12 µT sind, vorzugsweise nicht höher als 10 µT sind, insbesondere nicht höher als 4 - 7 µT sind.

Das Grundsignal besteht aus einer Folge von Einzelimpulsen mit einer Impulsbreite von ca. 33 ms in der angegebenen Höhe von 3-15 µT, beispielsweise von 6,5 µT und zwar für den vorgegebenen Zeitraum von 12-16 s. Danach folgt ein Zusatzimpuls mit einer Impulsbreite von 100-200 ms mit einer Intensität (Intensität = Impulsstärke = elektromagnetische Flussdichte) von 30-120 mT beispielsweise mit 70 µT.

Der Begriff "Zusatzsignal" im Sinne der Erfindung bedeutet somit, dass zu einem bestimmten Zeitpunkt für einen sehr kurzen Zeitraum, hier 100-200 ms, ein deutlich stärkeres Signal mit einer Intensität von wenigstens 18 µT höher als das Grundsignal zusätzlich abgegeben wird, wobei für den kurzen Zeitraum das Grundsignal von dem Zusatzsignal überlagert wird. Die Intensität des Zusatzsignals beträgt vorzugsweise 50 - 110 µT, insbesondere 60 - 100 µT.

Die Impulsfolgen bestehen aus Einzelimpulsen, deren Amplituden z.B. einer Exponentialfunktion folgen. Eine bevorzugte Exponentialfunktion ist in der EP 0995463 B1 beschrieben mit y = x³ · e^{sin(x³)}, wobei die Formel den Verlauf der Amplitude y über die Zeit x angibt. Die Einzelimpulse haben dann einen Verlauf etwa wie in Fig. 2 der EP 0995463 B1. Die Einzelimpulse können auch nicht-exponentielle Verläufe aufweisen, indem sie an- und absteigende Hüllkurven darstellen mit harmonischem oder anharmonischem Schwingungsverlauf wie in der WO 2008/025731. Alternierende Impulsgruppen mit derartigen Schwingungsverläufen sind z.B. in Fig. 4c bis 4f in der WO 2008/025731 wiedergegeben. Insgesamt bestehen die Impulsfolgen aus zusammengesetzten Schwingungen mit bogenförmigem Verlauf.

Impulse oder Impulsgruppen mit stufenweisem oder quadratischem Verlauf sind nicht Gegenstand der Erfindung.

Impulsgruppen mit Einzelimpulsen, deren Amplitude einer e-Funktion entspricht, sind für die vorliegende Erfindung bevorzugt.

"Mehrmalige Wiederholung" der Impulsfolgen bedeutet entsprechend der Steuerzeit eine 110- 240-malige Wiederholung.

In dem erzeugten elektromagnetischen Feld werden vorteilhaft durch die Frequenz, die Höhe des Grundsignals, die Höhe und Länge des Zusatzsignals und die Häufigkeit der Wiederholungen der Impulsfolgen funktionelle Merkmale der körpereigenen Regulation des Organismus beeinflusst - ausgewählt unter mikrozirkulatorischen Funktionsmerkmalen, makrozirkulatorischen Funktionsmerkmalen und immunologischen Funktionsmerkmalen.

Zu den mikrozirkulatorischen Funktionsmerkmalen gehören
- die Anzahl der blutzellperfundierten Knotenpunkte (nNP)
- die Änderungen des venulären Strömungsflusses (ΔQᵥₑₙ)
- die Änderungen der venolenseitigen Sauerstoffausschöpfung (ΔpO₂)
- der arterioläre bzw. venuläre Vasomotionszustand (A_{VM})
- die Anzahl adhärierender weißer Blutzellen an einer definierten Venoleninnenwand nWBC/A.

Zu den weiteren Merkmalen gehören
- die Laktat-Abflutung cLac (z.B. der Skelettmuskulatur)
- der Strömungsfluss der initialen Lymphe QL
- die Auswurffraktion der linken Herzkammer EF
- die Ausscheidungsfunktion der Nieren während des Schlafes und/oder der Relaxation.

Zu letzteren gehören z.B. harnpflichtige Substanzen, pH-Wert und Osmolalität des Urins und andere.

Die mikrozirkulatorischen Funktionsmerkmale betreffend wird bei der Anzahl der aktuell blutzellperfundierten Knotenpunkte in einem definierten mikrovaskulären Netzwerk, nNP, die Anzahl der blutzellperfundierten Verzweigungsorte in diesem Netzwerk als Maß für den Verteilungszustand des Blutes herangezogen. Als Grenzströmungsgeschwindigkeit der roten Blutzellen wird v_{RBC} = 80 µm/s definiert. Die Auswertung erfolgt in + oder - (verglichen mit dem definierten Ausgangswert n=60).

Mittels der vorliegenden Erfindung wird beispielsweise der Wert nNP mit der erfindungsgemäßen Impulsfolge, wiederholt über 8 Stunden, um 10 - 15 % gegenüber dem Anfangswert erhöht.

Der venuläre Strömungsfluß Qᵥₑₙ und der arterioläre Strömungsfluss Qₐᵣₜ ist der Teilchenstrom (Blutzell-Strom) in definierten Venolen bzw. Arteriolen. Er wird in µm³/s angegeben. ΔQᵥₑₙ ist die Änderung des venulären Strömungsflusses und kann im Vergleich mit dem Ausgangswert auch als prozentuale Änderung angegeben werden.

Die venolenseitige Sauerstoffausschöpfung ΔpO₂ wird als prozentuale Änderung im Vergleich mit dem jeweiligen Ausgangswert zum Zeitpunkt t=0 angegeben. Bestimmt wird die absolute Differenz der Sauerstoffsättigung des Hämoglobins in den zuführenden Arteriolen und abführenden Venolen des Netzwerkes eines ausgewählten Gewebe-Targets. Als Target werden Gewebeabschnitte von Haut oder Darm ausgewählt, die über ausgedehnte, reich verzweigte und kreislaufrepräsentative Blutgefäß-Netzwerke des Organismus verfügen und ferner zu den immunologisch aktiven Organen zählen, und die weiterhin für nichtinvasive Messungen leicht zugänglich sind.

Mittels der vorliegenden Erfindung wird beispielsweise der Wert ΔpO₂ mit einer Impulsfolge im Rahmen der Erfindung, die über 8 Stunden wiederholt wird, um 8 - 10 % erhöht.

Der spontane arterioläre (bzw. venuläre) Vasomotionszustand, A_{VM}, wird ermittelt, indem das Weg-Zeit-Diagramm der autorhythmischen Kontraktionsbewegungen glatter Muskelzellen der arteriolären Gefäßwand bestimmt wird (Messung der Distanz normal zur Mikrogefäß-Längsachse von Endotheloberfläche zu gegenüberliegender Endotheloberfläche zu äquidistanten Messzeitpunkten; pro Sekunde 60 Messwerte; Ermittlung der zusammengesetzten Schwingung; FOURIER-Analyse; Ermittlung des Amplituden-Frequenz-Spektrums). Kriterium ist der Flächeninhalt A unter der Einhüllenden des Amplituden-Frequenz-Spektrums der arteriolären Vasomotion (eine zusammengesetzte Schwingung). Angegeben wird der Wert als prozentuale Veränderung im Vergleich mit den Ausgangswerten.

Die Anzahl der adhärierenden weißen Blutzellen an einer definierten Venoleninnenwand, nWBC/A, wird an der definierten Innenfläche der Venole mit A = 18000 µm² gemessen. Gezählt werden alle weißen Blutzellen, die länger als 20 Sekunden am Endothel anhaften.

Mittels der vorliegenden Erfindung wird beispielsweise der Wert nWBC/A um 12 % erhöht bei einer Impulsfolge im Rahmen der Erfindung, die über 8 Stunden wiederholt wird. Der Vergleichswert zeigt weniger als 4 %.

Die mit der erfindungsgemäßen Vorrichtung und dem damit erzeugten amplituden- und frequenzmodulierten pulsierenden elektromagnetischen Feld hervorgerufenen Stimulierungseffekte betreffen lokale Regulationsmechanismen der Mikrozirkulation des Blutes, wie die spontane arterioläre Vasomotion unter besonderer Bevorzugung immunologisch aktiver Organe wie Haut und Darm, sowie damit im Zusammenhang die endothelvermittelte Tonusregulation kleinster muskelbewehrter Mikrogefäße.

Hinsichtlich der Wirkung bei lokalen Regulationsmechanismen wurde gefunden, dass mittels der erfindungsgemäßen Vorrichtung mit dem erzeugten pulsierenden elektromagnetischen Feld in der ganz speziellen Impulsfolge, Impulshöhe und Impulsdauer gegenüber bekannten elektromagnetischen Feldern höhere Beträge von Merkmalsänderungen erreicht werden, insbesondere 9 - 20 % höhere Merkmalsänderungen.

Dies ist besonders überraschend, weil Impulse mit Grund- und Zusatzsignal in höheren Frequenzbereichen diese Ergebnisse nicht zeigen.

Ein besonderer Vorteil der Erfindung besteht darin, dass die mehrmaligen Wiederholungen der Impulsfolgen über mehrere Stunden nachts während des Schlafes der Patienten ablaufen können und damit kein zusätzlicher Aufwand verbunden sein muss.

Weiterhin ergeben sich durch die Erfindung prophylaktische und komplementärtherapeutische Wirkungen, die viele Vorgänge der gesamt-organismischen Homöostase-Regulation betreffen, wie z.B. die Ausscheidungsfunktion der Nieren während der Nachtruhe.

Ein weiterer Gegenstand der Erfindung ist die Bereitstellung eines prophylaktischen oder therapeutischen Verfahrens zur Stimulierung autoregulativer lokaler und übergeordneter Mechanismen der Homöostase während der Schlafphase und der Ruhe-/Entspannungsphase des Organismus, dadurch gekennzeichnet, dass der Körper oder ein Teil des Körpers eines Patienten einem pulsierenden elektromagnetischen Feld ausgesetzt wird, wobei eine Impulsfolge die Folgen 1 bis 5 einschließt:
Folge 1: 0 µT für 1-3 Sekunden,
Folge 2: 3-12 ist Grundsignal für 12-16 Sekunden,
Folge 3: 30-150 ist Zusatzsignal für 100-200 Millisekunden,
Folge 4: 8- bis 10-malige Wiederholung der Folgen 2 und 3,
Folge 5: 0 µT für 1-3 Sekunden
mit folgenden Maßgaben
- die Frequenz für das Grundsignal und das Zusatzsignal ist gleich und liegt im Bereich von 8 bis 15 Hz
- die Impulsfolgen sind derart eingerichtet, dass sie mehrfach wiederholt werden und dabei die Steuerzeit aller mehrfach wiederholten Impulsfolgen auf wenigstens 3,5 Stunden innerhalb eines Zeitraumes von 7-9 Stunden bemessen ist
- die Amplituden der Einzelimpulse folgen einer Exponentialfunktion oder an- und absteigenden Hüllkurven mit harmonischem oder anharmonischem Verlauf.

Die Zeitdauer der Behandlung geht von einer einmaligen Anwendung bis zu einer täglichen Behandlung für 2 bis 90 Tage, wobei Unterbrechungen von 2-5 Tagen vorgesehen sein können.

Bei einer 7- bis 9-stündigen Behandlung in der Schlafphase eines Patienten kann die erfindungsgemäße Impulsfolge fortlaufend in diesem Zeitraum abgegeben werden. Eine weitere vorteilhafte Ausführungsform besteht in einer 2-stündigen Impulsfolge, 4 Stunden Pause und eine weitere 2-stündige Impulsfolge. Eine weitere vorteilhafte Ausführungsform besteht in halbstündigen Impulsfolgen und dazwischen halbstündigen Pausen über insgesamt 7 - 9 Stunden.

Eine vorteilhafte Gesamtsteuerzeit der Impulsfolgen beträgt 8 Stunden.
Diese und weitere vorteilhafte Ausführungsformen können in Abhängigkeit von Alter, Konstitution, Kondition und Zustand des zu Behandelnden vorgesehen werden.

Ein weiterer Gegenstand der Erfindung ist die Verwendung einer Vorrichtung zur Stimulierung lokaler und übergeordneter Mechanismen der Homöostase während der Schlafphase und der Ruhe- bzw. Entspannungsphase des Organismus, umfassend einen Impulsgenerator und eine Felderzeugungseinrichtung, wobei der Impulsgenerator so ausgestaltet ist, dass er zusammen mit der Felderzeugungseinrichtung ein pulsierendes elektromagnetisches Feld erzeugt durch Abgabe von Impulsfolgen, wobei eine Impulsfolge die Folgen 1 bis 5 einschließt:
Folge 1: 0 µT für 1-3 Sekunden,
Folge 2: 3-12 µT Grundsignal für 12-16 Sekunden,
Folge 3: 30-150 µT Zusatzsignal für 100-200 Millisekunden,
Folge 4: 8- bis 10-malige Wiederholung der Folgen 2 und 3,
Folge 5: 0 µT für 1-3 Sekunden
mit folgenden Maßgaben
- die Frequenz für das Grundsignal und das Zusatzsignal ist gleich und liegt im Bereich von 8 bis 15 Hz
- die Impulsfolgen sind derart eingerichtet, dass sie mehrfach wiederholt werden und dabei die Steuerzeit aller mehrfach wiederholten Impulsfolgen auf wenigstens 3,5 Stunden innerhalb eines Zeitraumes von 7-9 Stunden bemessen ist
- die Amplituden der Einzelimpulse folgen einer Exponentialfunktion oder an- und absteigenden Hüllkurven mit harmonischem oder anharmonischem und insgesamt bogenförmigemVerlauf.

Die Erfindung soll nachstehend durch Beispiele näher erläutert werden. In der dazugehörigen Zeichnung zeigen
- Fig. 1: Diagramm zum Merkmal nNP im Targetgewebe Subkutis, Intestinum nach Anwendung der erfindungsgemäßen Vorrichtung
- Fig. 2: Diagramm zum Merkmal ΔpO₂ im Targetgewebe Intestinum nach Anwendung der erfindungsgemäßen Vorrichtung
- Fig. 3: Diagramm zum Merkmal nWBC/A im Targetgewebe Subkutis, Intestinum nach Anwendung der erfindungsgemäßen Vorrichtung
- Fig. 4: Diagramm zum Merkmal Strömungsfluss der initialen Lymphe QL im Targetgewebe Subkutis, Intestinum nach Anwendung der erfindungsgemäßen Vorrichtung

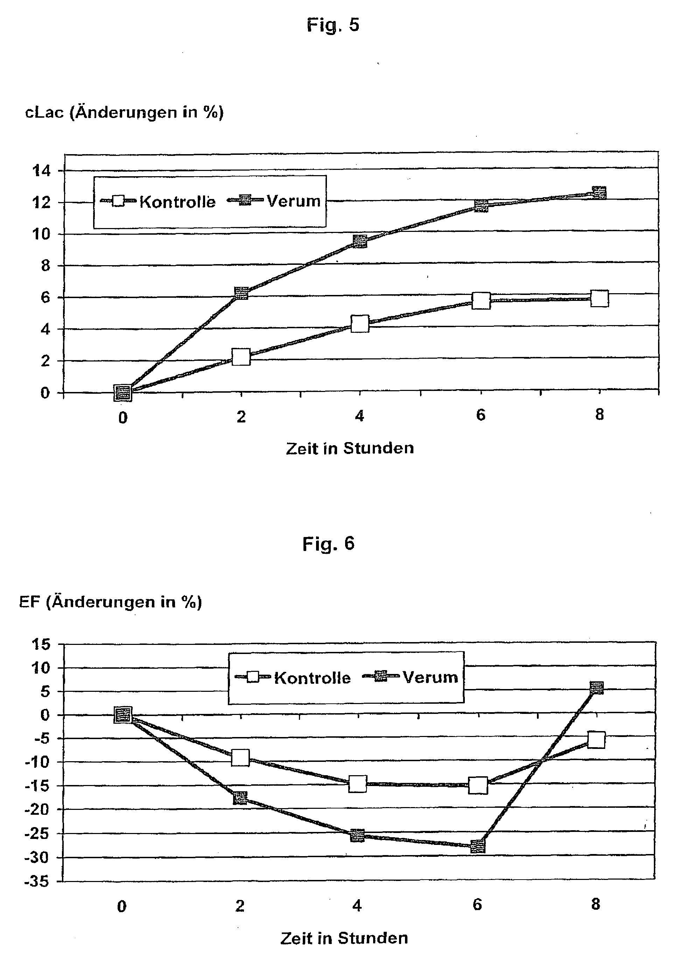

- Fig. 5: Diagramm zum Merkmal Lactat-Abflutung cLac im Targetgewebe Skelettmuskulatur nach Anwendung der erfindungsgemäßen Vorrichtung
- Fig. 6: Diagramm zum Merkmal Auswurffraktion der linken Herzkammer EF nach Anwendung der erfindungsgemäßen Vorrichtung.

### Beispiel 1

Die Vorrichtung besteht aus einem Impulsgenerator, der mit einer Netzspannung von 230 V Wechselstrom / 50 Hz gespeist wird, einer Steuereinrichtung für unterschiedlich hohe Feldstärken an den Applikatoren und einer Spulenmatte als Applikator. Die Betriebsspannung des Steuergerätes und damit die Nennleistung an der Spulenmatte beträgt maximal 12 V Gleichstrom. Eine bevorzugte Vorrichtung ist das Gerät BEMER der Firma Innomed International AG, Liechtenstein. Die Spulenmatte hat eine Fläche von 70x170 cm, so dass der gesamte oder nahezu gesamte liegende Körper eines Patienten darauf Platz hat. In der Spulenmatte sind drei Spulenpaare verteilt. Die elektromagnetische Flußdichte des elektromagnetischen Feldes sowie die zeitlichen Abstände werden über die Steuereinrichtung in mehreren Stufen gesteuert.

Die Vorrichtung mit dem erfindungsgemäß ausgestalteten Impulsgenerator wird bei einer Probandengruppe getestet.
Anzahl der Probanden: 24, aufgeteilt in eine Verum-Gruppe von 1.2 Probanden und eine Kontrollgruppe von 12 Probanden. Die Probanden waren männlich, Alter 44 - 55 Jahre, stressexponiert, mit Einschlaf- und Durchschlafstörungen, klinisch o.p.B.

Die Verum-Gruppe wurde wie folgt behandelt:
- liegend auf der Spulenmatte
- Impulsfolge (zusammengesetzte Schwingungen mit bogenförmigem Verlauf)

| | | | |
|---|---|---|---|
| Folge 1 | 0 µT | 2 s | |
| Folge 2 | 6,5 µT | 15 s | 10 Hz |
| Folge 3 | 80 µT | 120 ms | 10 Hz |
| Folge 4 | achtmalige Wiederholung von Folge 2 und 3; | | |
| Folge 5 | 0 µT | 2 s | |

Innerhalb des Grundsignals waren 4 Pausen von jeweils einer Sekunde eingeschaltet.
- Wiederholung der Impulsfolge für insgesamt 8 Stunden Behandlung
- Messung der Merkmale vom Zeitpunkt 0 bis 8 Stunden alle 2 Stunden (äquidistant)

Die Kontrollgruppe wurde wie folgt behandelt:
- liegend auf der Spulenmatte
- Impulsfolge: keine (Placebo)
- Messung wie Verum-Gruppe.

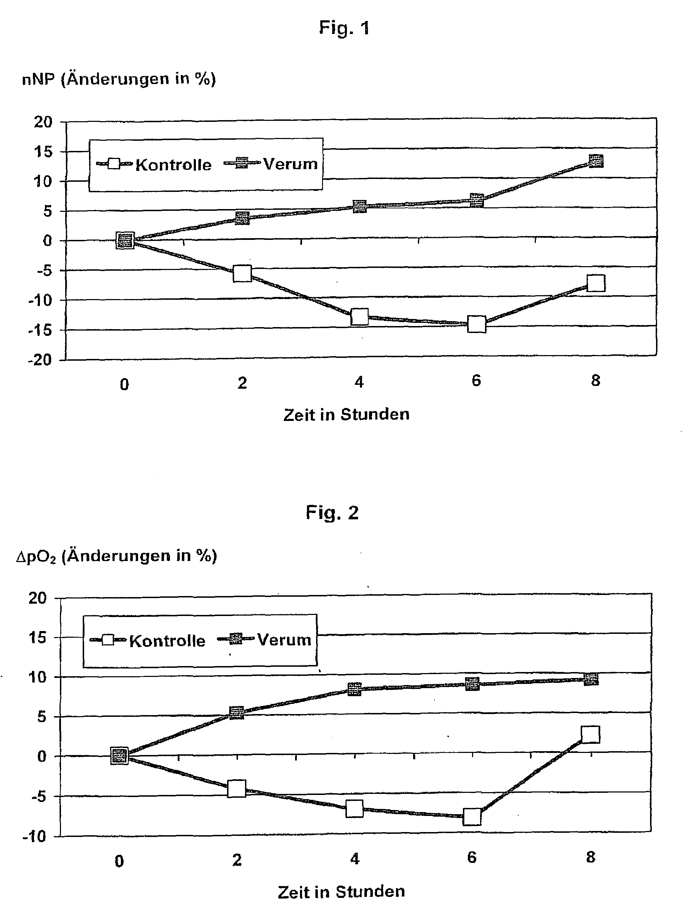

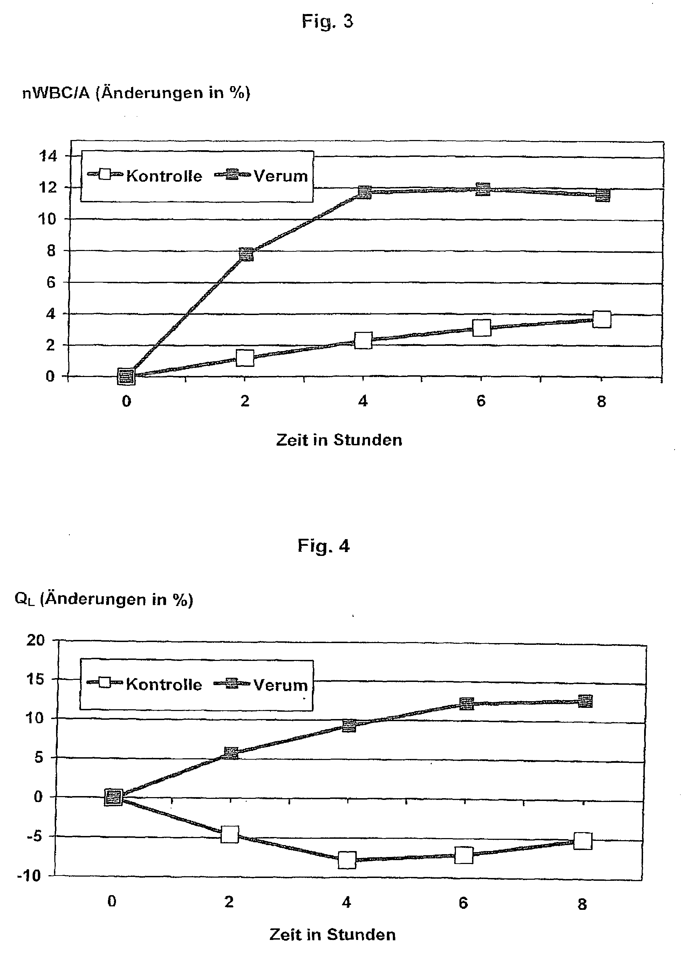

Die Behandlung erfolgte in beiden Gruppen an drei aufeinanderfolgenden Tagen. Als Meßmethoden wurden eingesetzt die Intravitalmikroskopie, Laser-DOPPLER-Mikroflußmessung, Weißlicht-Spektroskopie, intravitalmikroskopische Reflexionsspektroskopie und klinische Labordiagnostik. Von den untersuchten Merkmalen wurden hier ausgewählt:
1. Anzahl der blutzellperfundierten Knotenpunkte nNP in einer definierten Gewebeeinheit (V = 2000 µm³) im Targetgewebe Subkutis, Intestinum
   Aus der Darstellung in Fig. 1 ist zu entnehmen, dass gegenüber der Kontrollgruppe die Verum-Gruppe einen nahezu kontinuierlichen Anstieg während der Behandlung mit einer Änderung von 13 % zeigte. Der Wert der Kontrollgruppe fiel dagegen auf -15 % ab und erhöhte sich erst in den letzten beiden Stunden auf -8 %.
2. Venolenseitige Sauerstoffausschöpfung ΔpO₂ in der Mikrozirkulation einer definierten Gewebevolumeneinheit (V = 2000 µm³) im Targetgewebe Intestinum
   Aus der Darstellung in Fig. 2 ist zu entnehmen, dass gegenüber der Kontrollgruppe bei der Verum-Gruppe eine Erhöhung auf ein Maximum bei 9 % erfolgte. Bei der Kontrollgruppe gab es insgesamt keine Änderung, jedoch zwischenzeitlich nach 6 Stunden einen Abfall auf -8 %.
3. Anzahl adhärierender weißer Blutzellen aus einer definierten Venoleninnenwand nWBC/A in der Mikrozirkulation einer definierten Gewebevolumeneinheit (V = 2000 µm³) im Targetgewebe [Intestinum
   Aus der Darstellung in Fig. 3 ist zu entnehmen, dass gegenüber der Kontrollgruppe die Verum-Gruppe 8 Stunden nach der Behandlung eine deutlich erhöhte Änderung von über 11 % zeigte. Die Kontrollgruppe kam nur auf knapp 4 %.
4. Strömungsfluss der initialen Lymphe QL im Targetgewebe Subkutis, Intestinum Während die Kontrollgruppe eine Absenkung um -5 % erreichte, die zwischenzeitlich noch höher war, konnte die Verumgruppe einen Zuwachs von 13 % verzeichnen.
5. Laktat-Abflutung cLac im Targetgewebe Skelettmuskulatur
   Gegenüber einer Erhöhung bei der Kontrollgruppe auf etwa 5 % zeigte die Verumgruppe Merkmalserhöhungen von über 12 %.
6. Auswurffraktion der linken Herzkammer EF
   Die Kontrollgruppe erreichte nach Absenkung auf über -15 % einen Schlusswert nach 8 Stunden von etwa - 7 %. Die Veränderungen bei der Verumgruppe waren mit fast -30 % nach etwa 6 Stunden deutlich intensiver, erreichten jedoch nach 8 Stunden einen Wert von +5 %.

Insgesamt ergeben sich sowohl für Merkmale der Mikrozirkulation des Blutes als auch für andere Merkmale von Nierenfunktion (vermehrte Ausscheidung harn

pflichtiger Substanzen), stimulierte Immunreaktion in Haut und Darm während der Nachtruhe, deutliche Veränderungen mittels der erfindungsgemäßen Vorrichtung. Die Beträge sind um 9-20 % höher als zu erwarten.

### Beispiel 2 (Vergleichsbeispiel)

Es wird die Vorrichtung der vorliegenden Erfindung mit der Vorrichtung gemäß WO 2008/25731 verglichen.

Es wird mit einer Vorrichtung und einer Spulenmatte wie im Beispiel 1 gearbeitet.

Probanden: männlich, Alter 45 - 55 Jahre, im Rahmen eines physiotherapeutischen Konditionierungsprogrammes.

Verumgruppe 12 Probanden, Vergleichsgruppe 12 Probanden, ausgewählt durch Zufallsgenerator.

Behandlungsintervall 30 Tage (Behandlungen verblindet), Meßintervall ebenfalls 30 Tage (äquidistant und zur gleichen Uhrzeit).
(a) Behandlung der Verumgruppe, jeweils liegend auf der Spulenmatte mit der Impulsfolge (zusammengesetzte Schwingungen mit bogenförmigem Verlauf):

| | | | |
|---|---|---|---|
| Folge 1 | 0 µT | 2s | |
| Folge 2 | 8 µT | 12 s; | 12 Hz (Grundsignal) |
| Folge 3 | 100 µT | 150 ms | 12 Hz |
| Folge 4 | achtmalige Wiederholung von Folge 2 und 3 | | |
| Folge 5 | 0 µT | 2 s | |

Innerhalb des Grundsignals 3 Pausen von jeweils 1 s. Die genannte Impulsfolge 1 - 5 wird für insgesamt 7 Stunden wiederholt. Messungen erfolgen an den Tagen 0, 5, 10, 15, 20, 25 und 30.
(b) Behandlung der Vergleichsgruppe, jeweils liegend auf der Spulenmatte mit der Impulsfolge gemäß WO2008/026731 (zusammengesetzte Schwingungen mit bogenförmigem Verlauf):
Basisimpuls: 60 µT, Impulsbreite 30 ms
Zusatzimpuls: 180 µT, Impulsbreite 150 ms
Frequenz des Zusatzimpulses: 3 pro Minute
Gesamtfrequenz: 30 Hz
Behandlungsdauer: 2 x 25 Minuten im Abstand von 2 h
Behandlungsfolge: täglich für 30 Tage
Messungen an den Tagen 0, 5, 10, 15, 20, 25, 30

(c) Auswertungsmethoden
Vitalmikroskopische Untersuchungseinheit mit computergestützter Bildverarbeitung (System KONTRON), vitalmikroskopische Reflexionsspektometrie (System SPEX), kombinierte Laser-Doppler-Mikroflußmessung und Weißlichtspektroskopie (System LEA).
(d) Auswertungsmerkmale
Merkmale:
- Spontane arterioläre Vasomotion AVM (Flächeninhalt unter der Einhüllenden des originären Amplituden-Frequenzspektrums der kleinkalibrigen arteriolären Vasomotion),
- Anzahl der aktuell blutzellperfundierten Knotenpunkte im definierten Kapillarnetzwerk nNP,
- venolenseitige Sauerstoffausschöpfung ΔpO₂
- Strömungsfluß der initialen Lymphe QL.
Targetgewebe: Intestinum (Muscularis des Rectum).

### Biometrie :

WILCOXON-Rangsummentest (α = 5 %)

**Tabelle 1**

| Spontane arterioläre Vasomotion AVM | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 18,3 | 25,1 | 28,3 | 29,9 | 30,3 | 30,6 |
| Vergl.bsp. 2(b) WO2008/025731 | - | 6,2 | 14,6 | 15,2 | 15,5 | 15,6 | 15,6 |

**Tabelle 2**

| Anzahl der blutzellperfundierten Knotenpunkte nNP | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 15,1 | 22,3 | 27,0 | 31,8 | 33,5 | 34,9 |
| Vergl.bsp. 2(b) (WO2008/025731 | - | 4,9 | 9,6 | 13,9 | 15,2 | 15,6 | 15,7 |

**Tabelle 3**

| Venolenseitige Sauerstoffausschöpfung ΔpO₂ | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 15,1 | 25,2 | 30,6 | 35,0 | 36,6 | 37,9 |
| Vergl.bsp. 2(b) WO2008/025731 | - | 5,0 | 8,8 | 14,4 | 15,8 | 18,1 | 18,3 |

**Tabelle 4**

| Strömungsfluß der initialen Lymphe QL | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 20,8 | 29,2 | 34,8 | 40,4 | 42,0 | 44,2 |
| Vergl.bsp. 2(b) WO2008/025731 | - | 4,5 | 7,9 | 9,9 | 11,6 | 14,8 | 15,2 |

Der Vergleich zeigt signifikante Unterschiede für alle vier gemessenen Merkmale der Mikrozirkulation des Blutes zwischen der Vorrichtung des Standes der Technik WO 2008/025731 und der der Erfindung. Das Merkmal QL liegt teilweise um das 3-bis 4-fache höher, andere Merkmale um das 1,5- bis 2-fache.

## Patentansprüche

1. Vorrichtung zur Stimulierung autoregulativer lokaler und übergeordneter Mechanismen der Homöostase des Organismus, umfassend einen Impulsgenerator, ein Steuergerät und eine Felderzeugungseinrichtung, wobei der Impulsgenerator ausgestaltet ist, zusammen mit dem Steuergerät und der Felderzeugungseinrichtung ein pulsierendes elektromagnetisches Feld zu erzeugen durch Abgabe von Impulsfolgen, **dadurch gekennzeichnet, dass** eine Impulsfolge die Folgen 1 bis 5 einschließt:
Folge 1: 0 µT für 1-3 Sekunden,
Folge 2: 3-12 µT Grundsignal für 12-16 Sekunden,
Folge 3: 30-150 µT Zusatzsignal für 100-200 Millisekunden,
Folge 4: 8- bis 10-malige Wiederholung der Folgen 2 und 3,
Folge 5: 0 µT für 1-3 Sekunden
mit folgenden Maßgaben
- die Frequenz für das Grundsignal und das Zusatzsignal ist gleich und liegt im Bereich von 8 bis 15 Hz
- die Impulsfolgen sind derart eingerichtet, dass sie mehrfach wiederholt werden und dabei die Steuerzeit aller mehrfach wiederholten Impulsfolgen auf wenigstens 3,5 Stunden innerhalb eines Zeitraumes von 7-9 Stunden bemessen ist
- die Amplituden der Einzelimpulse folgen einer Exponentialfunktion oder bogenförmig an- und absteigenden Hüllkurven mit harmonischem oder anharmonischem Verlauf.

2. Vorrichtung nach Anspruch 1, **dadurch gekennzeichnet, dass** die Felderzeugungseinrichtung eine Spulenmatte ist.

3. Vorrichtung nach Anspruch 2, **dadurch gekennzeichnet, dass** in der Spulenmatte mehrere elektromagnetische Einzelspulen in gleichmäßiger Verteilung über die Gesamtfläche der Matte zur Erzeugung eines flächenhaften Magnetfeldes angeordnet sind.

4. Vorrichtung nach Anspruch 3, **dadurch gekennzeichnet, dass** die Einzelspulen als flächeninhaltsgleiche und/oder -unterschiedliche Leiterschleifen ausgebildet sind.

5. Vorrichtung nach einem der Ansprüche 1 bis 4, **dadurch gekennzeichnet, dass** in der Impulsfolge das Grundsignal 3-8 Pausen von 1-3 Sekunden mit 0 µT aufweist.

6. Vorrichtung nach einem der Ansprüche 1 bis 4, **dadurch gekennzeichnet, dass** in dem erzeugten pulsierenden elektromagnetischen Feld die Höhe des Grundsignals, die Höhe des Zusatzsignals und die Häufigkeit der Wiederholung der Impulsfolgen in Abhängigkeit von vorhandenen Werten gesteuert werden, ausgewählt unter
- Anzahl der blutzellperfundierten Knotenpunkte (nNP)
- Änderungen des venulären Strömungsflusses (ΔQᵥₑₙ)
- Änderungen der venolenseitigen Sauerstoffausschöpfung (ΔpO₂)
- arteriolärer bzw. venulärer Vasomotionszustand (A_{vM})
- Anzahl adhärierender weißer Blutzellen an einer definierten Venoleninnenwand nWBC/A
- Laktat-Abflutung cLac (z.B. der Skelettmuskulatur)
- Strömungsfluss der initialen Lymphe QL
- Auswurffraktion der linken Herzkammer EF
- Ausscheidungsfunktion der Nieren während des Schlafes und/oder der Relaxation.

## Claims

1. A device for stimulating autoregulative local and superordinate mechanisms of the homeostasis in the organism, comprising a pulse generator, a control device and a field generation means, wherein the pulse generator is configured to generate, together with the control device and the field generation means, a pulsating electromagnetic field by emitting pulse sequences, **characterized in that** a pulse sequence includes sequences 1 to 5:
sequence 1: 0 µT for 1 to 3 seconds,
sequence 2: 3 to 12 µT of basic signal for 12 to 16 seconds,
sequence 3: 30 to 150 µT of additional signal for 100 to 200 milliseconds,
sequence 4: 8- to 10-fold repetition of sequences 2 and 3,
sequence 5: 0 µT for 1 to 3 seconds
with the following requirements
- the frequency for the basic signal and the additional signal is identical and within the range of 8 to15 Hz
- the pulse sequences are set to be repeated multiple times, with the timing of all pulse sequences which are repeated multiple times set to be at least 3.5 hours within a period of 7 to 9 hours
- the amplitudes of the single pulses follow an exponential function or envelopes rising and falling in an arc-shaped manner with a harmonic or anharmonic progression.

2. The device according to claim 1, **characterized in that** the field generation means is a coil mat.

3. The device according to claim 2, **characterized in that** multiple single electromagnetic coils are arranged in the coil mat, uniformly distributed over the entire area of the mat in order to generate a planar magnetic field.

4. The device according to claim 3, **characterized in that** the single coils are designed as conductor loops having the same and/or differing areas.

5. The device according to any one of claims 1 to 4, **characterized in that** in the pulse sequence the basic signal has 3 to 8 pauses of 1 to 3 seconds with 0 µT.

6. The device according to any one of claims 1 to 4, **characterized in that** the level of the basic signal, the level of the additional signal and the frequency of repetition of the pulse sequences in the generated pulsating electromagnetic field are controlled as a function of available values, selected from
- number of the blood-cell-perfused nodal points (nNP)
- changes in the venular flow (ΔQᵥₑₙ)
- changes in the venular oxygen utilization (ΔpO₂)
- arteriolar or venular vasomotional state, respectively, (A_{VM})
- number of white blood cells adhering to a defined venule inner wall nWBC/A
- lactate clearance cLac (e.g. of the skeletal muscles)
- initial lymph flow QL
- ejection fraction of the left ventricle of the heart EF
- clearing function of the kidneys during sleep and/or relaxation.

## Revendications

1. Dispositif de stimulation de mécanismes autorégulateurs locaux et supérieurs de l'homéostasie de l'organisme, comprenant un générateur d'impulsions, un appareil de commande et un dispositif générateur de champs, le générateur d'impulsions étant constitué pour produire, conjointement avec l'appareil de commande et le dispositif générateur de champs, un champ électromagnétique pulsé par la délivrance de séquences d'impulsions, **caractérisé en ce qu'**une séquence d'impulsions inclut les séquences 1 à 5 :
Séquence 1: 0 µT pendant 1-3 secondes,
Séquence 2: 3-12 µT signal de base pendant 12-16 secondes,
Séquence 3: 30-150 µT signal supplémentaire pendant 100-200 millisecondes,
Séquence 4: 8 à 10 répétitions des séquences 2 et 3,
Séquence 5: 0 µT pendant 1-3 secondes
avec les conditions suivantes
- la fréquence pour le signal de base et le signal supplémentaire est identique et se situe dans la plage de 8 à 15 Hz
- les séquences d'impulsions sont agencées de telle sorte qu'elles sont répétées plusieurs fois, et la durée de commande de toutes les séquences d'impulsions répétées plusieurs fois est en l'occurrence dimensionnée à au moins 3,5 heures à l'intérieur d'une période de 7 à 9 heures
- les amplitudes des différentes impulsions suivent une fonction exponentielle ou des enveloppantes montantes et descendantes en forme d'arc avec une allure harmonique ou anharmonique

2. Dispositif selon la revendication 1, **caractérisé en ce que** le dispositif générateur de champs est un mat de bobines.

3. Dispositif selon la revendication 2, **caractérisé en ce que**, dans le mat de bobines, il est disposé plusieurs bobines individuelles électromagnétiques réparties régulièrement sur la surface totale du mat pour la production d'un champ magnétique plan.

4. Dispositif selon la revendication 3, **caractérisé en ce que** les bobines individuelles sont constituées en tant que boucles conductrices de même aire surfacique et/ou d'aire surfacique différente.

5. Dispositif selon une des revendications 1 à 4, **caractérisé en ce que**, dans la séquence d'impulsions, le signal de base présente 3 à 8 pauses de 1 à 3 secondes avec 0 µT.

6. Dispositif selon une des revendications 1 à 4, **caractérisé en ce que**, dans le champ électromagnétique pulsé produit, la hauteur du signal de base, la hauteur du signal supplémentaire et la fréquence de répétition des séquences d'impulsions sont commandées en fonction de valeurs existantes sélectionnées parmi
- le nombre de points nodaux perfusés avec des cellules du sang (nNP)
- les modifications du flux vénulaire (ΔQᵥₑₙ)
- des modifications de l'utilisation de l'oxygène côté veinules (ΔpO₂)
- état vasomoteur artériolaire ou respectivement vénulaire (A_{VM})
- nombre de globules blancs qui adhèrent sur une paroi intérieure de veinules définie nWBC/A
- élimination du lactate cLac (par ex. de la musculature du squelette)
- flux de la lymphe initiale QL
- fraction d'éjection ventriculaire gauche EF
- fonction d'excrétion des reins pendant le sommeil et/ou pendant la relaxation.
